# EUROPÄISCHE PATENTSCHRIFT

(11) **EP 3 083 053 B1**
(45) Veröffentlichungstag und Bekanntmachung des Hinweises auf die Patenterteilung: **18.12.2019**
(21) Anmeldenummer: 14824461.9
(22) Anmeldetag: 19.12.2014
(51) Int. Cl.: B01L 3/00, C12M 1/00, C12M 1/32, C12Q 1/686, G01N 35/00, G01N 35/10

(54) **ABDECKVORRICHTUNG, INSBESONDERE DECKEL FÜR DIE ABDECKUNG VON REAKTIONSGEFÄSSEN**
COVERING DEVICE, IN PARTICULAR LID, FOR COVERING REACTION VESSELS
DISPOSITIF DE RECOUVREMENT, EN PARTICULIER COUVERCLE SERVANT À COUVRIR DES RÉCIPIENTS RÉACTIONNELS

(30) Priorität: 20.12.2013 DE 102013114732
(43) Veröffentlichungstag der Anmeldung: 26.10.2016
(73) Patentinhaber: Hamilton Bonaduz AG, 7402 Bonaduz (CH)
(72) Erfinder: WOIZENKO, Eduard, 51503 Rösrath (DE); SFERLAZZA, Sandro, CH-7402 Bonaduz (CH)
(74) Vertreter: Müller Hoffmann & Partner
(86) Internationale Anmeldenummer: PCT/EP2014/078695
(87) Internationale Veröffentlichungsnummer: WO 2015/091928

(56) Entgegenhaltungen:
- EP-A2- 0 828 560
- EP-A2- 1 192 995
- US-A1- 2011 293 488
- US-A1- 2012 164 725

## Beschreibung

Die vorliegende Erfindung betrifft eine Abdeckvorrichtung, insbesondere Deckel, für die Abdeckung von Reaktionsgefäßen, insbesondere PCR-Platten, Mikrotiterplatten und dergleichen.

Aus der EP 1 142 795 A2 ist eine Abdeckmatte aus einem elastischen Material bekannt mit einer weichen unteren Lage, die zur Versteifung mit einer Trägerplatte versehen ist. Die Trägerplatte ist dabei derart gekrümmt, dass sie bei einer auf die gesamte Fläche der Trägerplatte wirkenden Druckkraft eine krümmungsfreie Form einnimmt, in der sie Abdeckmatte Vertiefungen in einem Reaktionsgefäß dicht verschließt. Lässt der Druck auf die Trägerplatte nach geht diese wieder in ihren gekrümmten Zustand zurück und hebet sich teilweise vom Reaktionsgefäß ab, so dass sie mit wenig Kraftaufwand vom Reaktionsgefäß entfernt werden kann.

Eine weitere Abdeckungsvorrichtung mit einer gekrümmten Trägerplatte und seitlich daran angebrachten gelenkig verbundenen Seitenwänden ist aus der EP 1 192 995 A2 bekannt. Bei einer auf die gekrümmte Trägerplatte wirkenden Druckkraft nimmt auch diese Trägerplatte eine krümmungsfreie Form ein und kann mittels der gelenkig an der Trägerplatte angebrachten Seitenwände an einem Reaktionsgefäß befestigt und in der krümmungsfreien Form gehalten werden.

Aus der WO 96/39481 ist eine Abdeckungsvorrichtung bekannt mit federnden Dichtrippen, die kreuzend über abzudichtenden Vertiefungen eines Probenbehälters angeordnet werden.

Die bekannten gekrümmten Trägerplatten mit den daran angebrachten weichen Dichtungsmatten haben den Nachteil, dass sie durch einen in den Vertiefungen der Reaktionsgefäße erzeugten Unterdruck an dem Reaktionsgefäß anhaften und nur teilweise von diesen gelöst werden, wenn Sie von der krümmungsfreien Form in die gekrümmte Form übergehen. Ein solches Anhaften der Abdeckvorrichtung ist in automatisierten Analyseverfahren, beispielsweise PCR-Verfahren, Nachweisverfahren für Viren oder Bakterien oder ähnliche Verfahren, wie sie für die Bioanalytik in Forschung, Diagnose und Forensik angewendet werden, unerwünscht. Das Anhaften von Abdeckvorrichtung an Reaktionsgefäßen kann zu einem Unterbruch eines gesamten automatisiert ablaufenden Verfahrens führen. Das Anhaften der Abdeckvorrichtung kann beim Lösen auch zu einem kurzzeitigen Anheben des Reaktionsgefäßes selbst führen mit dem Risiko, dass Probenflüssigkeit aus den Vertiefungen austreten kann und es somit zur gegenseitigen Kontamination von Proben kommen kann.

Aufgabe der Erfindung ist es, eine Abdeckvorrichtung bereitzustellen, welche die genannten Nachteile vermeidet und insbesondere vollständig vom Reaktionsgefäß entfernbar ist ohne Anhaften.

Zur Lösung wird eine Abdeckvorrichtung gemäß Anspruch 1 vorgeschlagen.

Eine solche Abdeckvorrichtung umfasst einen im Wesentlichen ebenen Hauptkörper mit einer Innenseite und einer Außenseite, wenigstens ein am Hauptkörper angeordnetes und mit diesem verbundenes, flächiges Dichtmittel, wobei das wenigstens eine Dichtmittel an der Innenseite des Hauptkörpers angeordnet ist,
einen dem Umfang des Hauptkörpers entlang verlaufenden Randabschnitt, der sich von der Außenseite in Richtung der Innenseite und über diese hinaus erstreckt, und
wenigstens ein am Hauptkörper oder am Dichtmittel angeordnetes Federelement, das derart ausgeführt ist, dass es in seinem entspannten Zustand die Abdeckvorrichtung auf einer der Abdeckvorrichtung zugewandten Oberfläche eines abzudeckenden Reaktionsgefäßes abstützt und dass zwischen dem Dichtmittel und der Oberfläche des Reaktionsgefäßes ein Zwischenraum gebildet ist.

Der entspannte Zustand des Federelements kann auch als Grundzustand des Federelements bezeichnet werden und wird von diesem eingenommen, wenn die Abdeckvorrichtung druckfrei auf dem Reaktionsgefäß angeordnet ist und hierdurch ein Abstand zwischen dem Dichtmittel und der Oberfläche des Reaktionsgefäßes vorliegt. Diese druckfreie Anordnung tritt in den Analyseverfahren üblicherweise vor und nach dem Anwenden von Reaktionsschritten auf, bei denen Vertiefungen im Reaktionsgefäß verschlossen sein müssen. Insbesondere tritt dieser Grundzustand direkt nach dem Absetzen der Abdeckvorrichtung auf dem Reaktionsgefäß und unmittelbar vor dem Entfernen der Abdeckvorrichtung vom Reaktionsgefäß auf. Da der Hauptkörper im Wesentlichen eben ausgebildet ist und nicht gekrümmt ist, kann durch das Federelement der gesamte Hauptkörper mit daran angeordnetem Dichtmittel im Abstand zum Reaktionsgefäß gehalten werden.

Das Federelement ist ferner derart ausgeführt, dass in seinem gespannten Zustand das Dichtmittel auf der Oberfläche des abgedeckten Reaktionsgefäßes aufliegt, um im Reaktionsgefäß angeordnete Probenbehälter, die vorzugsweise als Vertiefungen bzw. Kavitäten ausgebildet sind, dicht zu verschließen.

Wird Druck auf den Hauptkörper von dessen Außenseite her ausgeübt, kann die Abdeckvorrichtung unter Verformung und Vorspannung des Federelements gegen die Oberfläche des Reaktionsgefäßes gedrückt werden, um dessen Vertiefungen dicht zu verschließen. Vorzugsweise wird der Druck auf den Hauptkörper bzw. die Abdeckvorrichtung durch einen Gerätedeckel, der auch ein Heizelement umfassen kann, ausgeübt, wenn dieser Gerätedeckel geschlossen wird bzw. ist. Es kann sich dabei beispielsweise um den Gerätedeckel eines Thermoblocks oder eines Thermocyclers handeln.

Wird der auf den Hauptkörper bzw. die Abdeckvorrichtung wirkende Druck gelöst, entspannt sich das zuvor verformte und vorgespannte Federelement wieder und geht in seinen entspannten Zustand (Grundzustand) über, wodurch der ebene Hauptkörper gemeinsam mit dem daran angebrachten Dichtmittel vom Reaktionsgefäß angehoben wird, da sich das Federelement auf der Oberfläche des Reaktionsgefäßes abstützt. Anders ausgedrückt bewegt sich die Abdeckvorrichtung relativ zum Reaktionsgefäß aufgrund des sich entspannenden Federelements.

Das Federelement kann mit wenigstens einem Ende am Hauptkörper oder/und am Randabschnitt befestigt sein, vorzugsweise einstückig mit dem Hauptkörper oder/und dem Randabschnitt ausgebildet sein. Hierzu wird ferner vorgeschlagen, dass das Federelement wenigstens abschnittsweise gebogen ausgeführt ist mit wenigstens einer bezogen auf die Innenseite des Hauptkörpers konvexen oder/und konkaven Krümmung.

Das Federelement kann ferner als einseitig oder zweiseitig am Hauptkörper oder/und am Randabschnitt befestigter blattfederartiger Steg ausgebildet sein. Dabei kann der blattfederartige Steg einseitig am Randabschnitt oder am Hauptkörper befestigt sein mit einem beweglichen freien Ende.

Alternativ wird vorgeschlagen, dass der blattfederartige Steg zweiseitig am Hauptkörper oder am Dichtmittel befestigt ist und der Steg zwischen den beiden Befestigungsbereichen mehrere wellenartige Krümmungen aufweist.

Um eine Auslenkung des Federelements in seinem gespannten Zustand zu ermöglichen, wird vorgeschlagen, dass der Hauptkörper wenigstens eine dem wenigstens einen Federelement zugeordnete Federöffnung umfasst, die derart ausgebildet ist, dass sie im gespannten Zustand des Federelements dieses wenigstens teilweise aufnimmt, insbesondere dessen bewegliches freies Ende.

Es ist bevorzugt, dass die Abdeckvorrichtung wenigstens zwei Federelemente umfasst, vorzugsweise eine gerade Anzahl von Federelementen. Dabei können die Federelemente entlang des Umfangs des Hauptkörpers verteilt angeordnet sein. Das Vorsehen von zwei oder mehr, insbesondere von einer geraden Anzahl Federelementen ermöglicht eine gleichmäßige Abstützung der Abdeckvorrichtung auf dem Reaktionsgefäß. Ferner wirken die Vorspannkräfte der einzelnen Federelemente gemeinsam als eine gesamte Vorspannkraft auf die Abdeckvorrichtung, so dass diese nach Lösen des aufgebrachten Drucks gleichmäßig von einem abgedeckten Reaktionsgefäß angehoben werden kann. Eine gerade Anzahl von Federelementen bietet sich insbesondere Reaktionsgefäßen bzw. Abdeckvorrichtung an, die eine vieleckige, insbesondere rechteckige Grundform aufweisen. Bei einer anderen Grundform, wie etwa einem Kreis oder einem anderen Polygon (beispielsweise Sechseck) können auch drei Federelemente verwendet werden. Es wird in diesem Zusammenhang darauf hingewiesen, dass auch ein einzelnes Federelement ausreichend sein kann. Denkbar ist beispielsweise bei einer rechteckigen Grundform der Abdeckvorrichtung ein Federelement, dass entlang einer Seite angeordnet ist, so dass die Abdeckvorrichtung im entspannten Zustand des Federelements geneigt zur Oberfläche des Reaktionsgefäßes auf diesem aufliegt. Denkbar ist bei entsprechender Ausgestaltung des Reaktionsgefäßes auch ein einzelnes zentral angeordnetes Federelement.

Weiterbildend wird vorgeschlagen, dass der Randabschnitt einen sich am Hauptkörper anschließenden oberen Rand und einen mit dem oberen Rand verbundenen unteren Rand aufweist, wobei ein entlang des oberen Randes gemessener Umfang kleiner ist als der Umfang entlang des unteren Randes. Dabei können der obere Rand und der untere Rand durch einen stufenartigen umlaufenden Vorsprung miteinander verbunden sein, der zum oberen und zum unteren Rand geneigt verläuft, vorzugsweise im Wesentlichen orthogonal verläuft. Hierzu wird ferner vorgeschlagen, dass der untere Rand und der stufenartige Vorsprung derart dimensioniert sind, dass die Abdeckvorrichtung auf einer weiteren gleichartigen Abdeckvorrichtung stapelbar ist, wobei im gestapelten Zustand die Abdeckvorrichtung mit ihrem unteren Rand auf dem stufenartigen Vorsprung der darunter angeordneten weiteren Abdeckvorrichtung aufliegt.

Ein in der genannten Art ausgebildeter Randabschnitt mit stufenartig zueinander angeordneten Rändern ermöglicht ein einfaches, reibungsarmes und klemmfreies Stapeln von Abdeckvorrichtung. Das Stapeln der Abdeckvorrichtungen ist insbesondere beim Verpacken derselben von Bedeutung, das die Packungsdichte erhöht werden kann. Ferner benötigt ein Stapel von Abdeckvorrichtungen mit derartigen Randabschnitten auch weniger Platz in einer Analyse- bzw. Dosiervorrichtung zum automatisierten Durchführen von Analyseverfahren.

Um die automatisierte Verwendung der Abdeckvorrichtung zu verbessern wird ferner vorgeschlagen, dass im Randabschnitt, vorzugsweise im oberen Rand, Kopplungsmittel vorgesehen sind, um die Abdeckvorrichtung mittels einer Greifvorrichtung lösbar aufzunehmen und zu transportieren, wobei die Kopplungsmittel vorzugsweise als Öffnungen im Randabschnitt ausgebildet sind, in die korrespondierende Nocken einer Greifvorrichtung einführbar sind.

Der Hauptkörper weist zwei parallele Längsseiten und zwei parallele Querseiten auf, wobei die Federelemente entlang der Längsseiten oder/und entlang der Querseiten angeordnet sind. Die Federelemente können entlang der Längsseiten oder/und entlang der Querseiten regelmäßig verteilt sein. Dabei können entlang der beiden Längsseiten beispielsweise anders ausgebildete bzw. geformte Federelemente eingesetzt werden als entlang der beiden Querseiten. Ferner kann auch die Anzahl der Federelemente entlang der Längsseiten und entlang der Querseiten unterschiedlich sein.

Um eine gewünschte Steifigkeit des ebenen Hauptkörpers und eine ermüdungsfreie Vorspannung der Federelemente der Abdeckvorrichtung zu erreichen, wird vorgeschlagen, dass der Hauptkörper, der Randabschnitt und die Federelemente aus einem Kunststoff hergestellt sind, vorzugsweise aus einem spritzgussfähigen Kunststoff, wie etwa Polycarbonat (PC), Polymerblends (ASB-PC), Polyamid (PA) oder Polybutylenterephtalat (PBT), wobei der Hauptkörper, der Randabschnitt und die Federelemente vorzugsweise einstückig ausgebildet sind. Das Dichtmittel kann aus einem thermoplastischen Elastomer (TPE) oder einem silikonhaltigen Elastomer hergestellt sein.

Die Dicke des Hauptkörpers zwischen seiner Außenseite und seiner Innenseite beträgt bevorzugt etwa 0,5 bis 3,0 mm, insbesondere etwa 1,0 bis 2,0 mm. Hierdurch wird eine ausreichende Steifigkeit des Hauptkörpers erreicht. Die Dicke des Dichtmittels beträgt bevorzugt etwa 0,5 bis 3,0 mm, insbesondere etwa 1,0 bis 2,0 mm. Hierdurch wird eine zuverlässige Abdichtung der Vertiefungen des Reaktionsgefäßes erreicht, wenn die Abdeckvorrichtung auf das Reaktionsgefäß gedrückt wird.

Die Erfindung betrifft ferner ein Reaktionsgefäß, insbesondere PCR-Platte oder Mikrotiterplatte, umfassend mehrere Vertiefungen bzw. Kavitäten als Probenbehälter, wobei das Reaktionsgefäß mit einer Abdeckvorrichtung, die wenigstens eines der oben beschriebenen Merkmale umfasst, abgedeckt ist, wobei vorzugsweise das Reaktionsgefäß nach den ANSI-Standards ANSI/SLAS 1-2004 bis ANSI/SLAS 4-2004 ausgeführt ist. Bevorzugt handelt es sich also um Mikrotiterplatten mit einer Dimension von , wobei darin 96, 384 .... Vertiefungen bzw. Kavitäten bzw. Näpfchen (auch als Wells bezeichnet) vorhanden sind, in denen Probenflüssigkeit aufgenommen werden kann.

Die Erfindung betrifft gemäß Anspruch 14 ferner auch ein Verfahren zum Abdecken von Reaktionsgefäßen, umfassend die Schritte:
a) Bereitstellen eines Stapels mit mehreren Abdeckvorrichtungen, die wenigstens ein oben beschriebenes Merkmal umfassen;
b) Bereitstellen von wenigstens einem abzudeckenden Reaktionsgefäß, insbesondere einer PCR-Platte oder einer Mikrotiterplatte;
c) Ergreifen einer obersten Abdeckvorrichtung des Stapels mittels einer Greifvorrichtung;
d) Bewegen der ergriffenen Abdeckvorrichtung zu dem bzw. zu einem abzudeckenden Reaktionsgefäß;
e) Absetzen der ergriffenen Abdeckvorrichtung auf dem Reaktionsgefäß;
f) Druck ausüben auf die Abdeckvorrichtung auf dem Reaktionsgefäß unter Verformung und Vorspannung der Federelemente der Abdeckvorrichtung, um Vertiefungen im Reaktionsgefäß mittels des Dichtmittels dicht zu verschließen;
g) Durchführen von für ein gewünschtes Analyseverfahren erforderlichen Schritten mit verschlossenem Reaktionsgefäß, wie etwa Temperatur erhöhen oder/und senken;
h) Freigabe der Abdeckvorrichtung durch Nachlassen des aufgebrachten Drucks unter Rückverformung und Entspannung der Federelemente, um das Dichtmittel vollständig von dem Reaktionsgefäß zu lösen;
i) Ergreifen der gebrauchten Abdeckvorrichtung mittels einer Greifvorrichtung und Entsorgen der Abdeckvorrichtung.

Beim Verfahren können die Schritte a) bis e) wiederholt ausgeführt werden, so dass Nacheinander mehrere Reaktionsgefäße mit jeweiligen Abdeckvorrichtungen abgedeckt werden, wobei die Schritte f) bis h) vorzugsweise gleichzeitig für alle abgedeckten Reaktionsgefäße durchgeführt werden und wobei vorzugsweise der Schritt i) wiederholt durchgeführt wird bis alle verwendeten Abdeckvorrichtungen von den jeweiligen Reaktionsgefäßen entfernt sind.

Es ist beim Schritt i) ferner denkbar, dass auch dieser Schritt das Stapeln der gebrauchten Abdeckvorrichtungen umfasst, damit die Packungsdichte des zu entsorgenden Materials möglichst hoch ist.

Insgesamt ist das Verfahren auf eine hoch automatisierte Verwendung von Abdeckungsvorrichtungen im Rahmen von automatisierten Analyseverfahren ausgerichtet, wobei die oben vorgestellte Abdeckvorrichtung mit den Federelementen einen wesentlichen Betrag zur zuverlässigen Automatisierung beiträgt. Ferner dient auch die durch die Randgestaltung erreichte einfache Stapelbarkeit der Abdeckungsvorrichtungen der verbesserten Automatisierung.

Das Verfahren wird vorzugsweise durch eine automatisierte Dosiervorrichtung, insbesondere einen Pipettierroboter durchgeführt.

Schließlich betrifft die Erfindung auch eine Dosiervorrichtung gemäß Anspruch 16, insbesondere Pipettierroboter, umfassend:
eine Auflageplatte, auf welcher ein Stapel aus nicht gebrauchten Abdeckvorrichtungen mit wenigstens einem der oben genannten Merkmale positioniert ist und auf welcher wenigstens ein Reaktionsgefäß positioniert ist, und
eine relativ zur Aufnahmeplatte in drei Hauptrichtung bewegliche Greifvorrichtung, die dazu eingerichtet ist, Abdeckvorrichtungen vom Stapel zu ergreifen und auf dem Reaktionsgefäß abzusetzen, sowie eine gebrauchte Abdeckvorrichtung von einem Reaktionsgefäß zu entfernen und zu entsorgen.

Nachfolgend wird die Erfindung unter Bezugnahme auf die anliegenden Figuren beispielhaft und nicht einschränkend beschrieben.
- Fig. 1: zeigt schematisch und vereinfacht eine erste Ausführungsform einer Abdeckvorrichtung in einer Aufsichtsdarstellung, in zugehörigen Querschnitten entsprechend den Schnittlinien A-A und B-B und vergrößerten Schnittbereichen I und II und in einer seitlichen Aufrissdarstellung.
- Fig. 2: zeigt zeigt schematisch und vereinfacht die erste Ausführungsform einer Abdeckvorrichtung mit einem darunter angeordneten Reaktionsgefäß in einer Aufsichtsdarstellung, einer Schnittdarstellung entsprechend der Schnittlinie C-C sowie zwei seitlichen Aufrissdarstellungen einer Längsseite und einer Querseite.
- Fig. 3: zeigt in den Teilfiguren a) und b) Querschnitte entsprechend der Schnittlinie C-C der Fig. 2, wobei Teilfigur a) die Abdeckvorrichtung mit entspannten Federelementen auf dem Reaktionsgefäß zeigt und wobei Teilfigur b) die Abdeckvorrichtung mit gespannten Federelementen auf dem Reaktionsgefäß zeigt, und zugehörige Schnittvergrößerungen III und IV.
- Fig. 4: zeigt in einer perspektivischen Ansicht die Abdeckungsvorrichtung der ersten Ausführungsform zusammen mit dem Reaktionsgefäß.
- Fig. 5: zeigt schematisch und vereinfacht einen Stapel von Abdeckungsvorrichtungen in seitlicher Aufrissdarstellung.
- Fig. 6: zeigt schematisch und vereinfacht eine zweite Ausführungsform einer Abdeckvorrichtung in einer Aufsichtsdarstellung, in einem zugehörigen Querschnitt entsprechend der Schnittlinie D-D und einem vergrößerten Schnittbereich V.
- Fig. 7: zeigt schematisch und vereinfacht eine dritte Ausführungsform einer Abdeckvorrichtung in einer Aufsichtsdarstellung

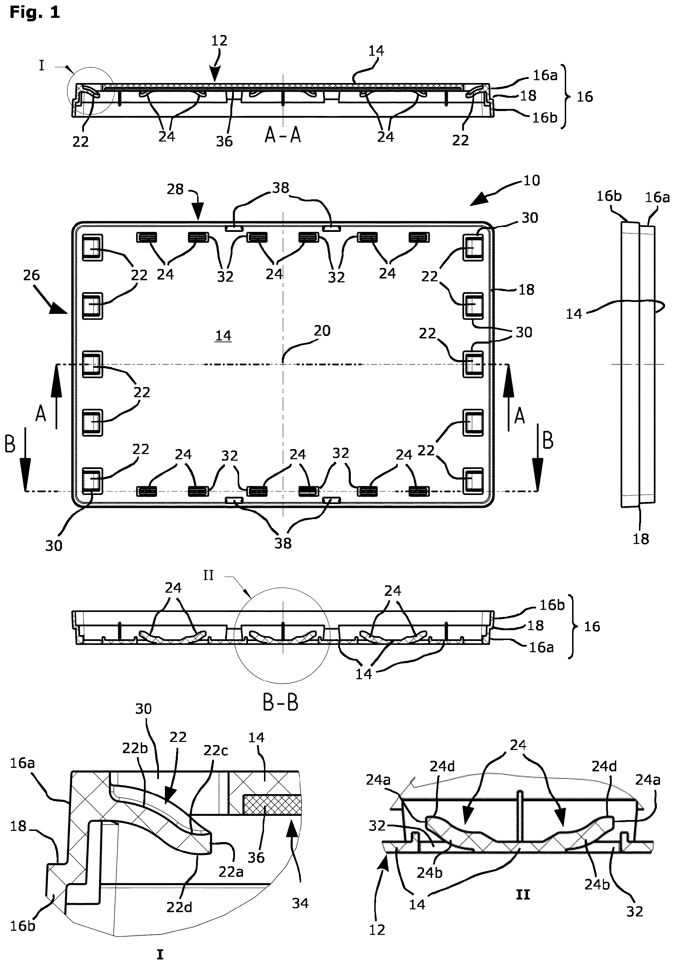

In Fig. 1 ist eine Abdeckvorrichtung 10 einer ersten Ausführungsform in Aufsichtsdarstellung mit Blickrichtung auf die Außenseite 12 und zugehörigen Schnittdarstellungen entsprechend der Schnittlinien A-A, B-B und Vergrößerungen I und II gezeigt. Ferner ist eine seitliche Aufrissdarstellung gezeigt. Die Beschreibung der Abdeckvorrichtung 10 erfolgt unter gleichzeitiger Bezugnahme auf die unterschiedlichen Darstellungen, ohne dass diese jedes Mal explizit erwähnt werden.

Die Abdeckvorrichtung umfasst einen Hauptkörper 14 und einen Randabschnitt 16 mit einem oberen Rand 16a und einem unteren Rand 16b. Die beiden Ränder 16a und 16b sind über einen Vorsprung bzw. eine Stufe 18 miteinander verbunden, wobei der untere Rand 16b einen größeren Abstand zu einem Zentrum 20 der Abdeckvorrichtung 10 aufweist als der obere Rand 16a. Anders ausgedrückt weist der obere Rand 16a einen kleineren Umfang auf als der untere Rand 16b.

Die Abdeckvorrichtung 10 umfasst mehrere Federelemente 22 und 24, wobei die Federelemente 22 entlang der Querseiten 26 und die Federelemente 24 entlang der Längsseiten 28 der Abdeckvorrichtung vorgesehen sind. In der Draufsicht und den Schnittdarstellungen sind ferner Federöffnungen 30 und 32 ersichtlich, die im Hauptkörper 14 ausgebildet sind. Der Hauptkörper 14 weist auf seiner Innenseite 34 ein Dichtmittel 36 auf, das bevorzugt aus einem weicheren Material als der Hauptkörper 14 hergestellt ist.

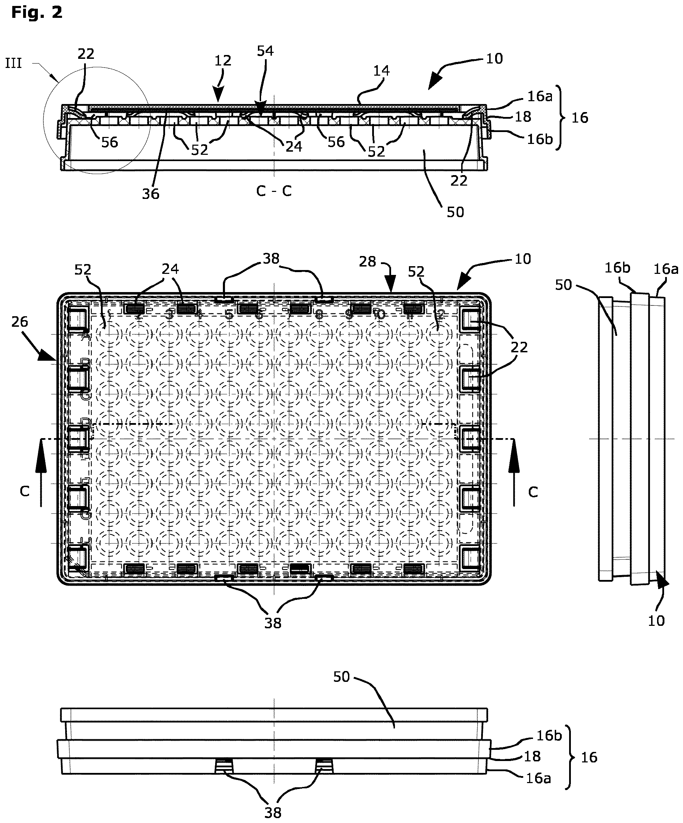

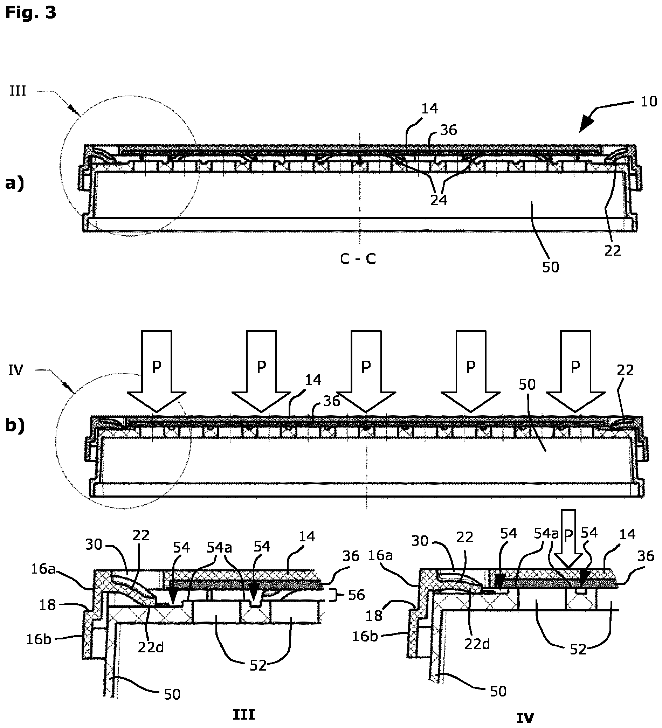

Die Federelemente 22 sind entlang der Querseiten 26 verteilt angeordnet und am Randabschnitt 16, insbesondere am oberen Rand 16a befestigt bzw. einstückig mit diesem ausgebildet (Schnittvergrößerung I). Das Federelement 22 ist somit im Bereich des Randabschnitts 16 in der Art einer Einspannung befestigt und weist ein bewegliches freies Ende 22a auf. Das Federelement 22 kann als gebogener, insbesondere blattfederartiger Steg ausgebildet sind mit bezogen auf die Innenseite 34 des Hauptkörpers 14 konvexen (22b) und konkaven (22c) Krümmungen. Das Federelement 22 umfasst ferner einen Auflagebereich 22d, der zur Abstützung der Abdeckvorrichtung 10 auf einem Reaktionsgefäß 50 dient, was später unter Bezugnahme auf die Figuren 2 und 3 noch beschrieben wird.

Die Federelemente 24 sind entlang der Längsseiten 28 verteilt angeordnet und am Hauptkörper 14 befestigt bzw. einstückig mit diesem ausgebildet (Schnittvergrößerung II). Die Federelemente 24 weisen ebenfalls bewegliche freie Enden 24a und sind in der Art einer Einspannung mit dem Hauptkörper 14 verbunden. Ferner weisen die Federelemente 24 Auflagebereiche 24d auf, die zur Abstützung der Abdeckvorrichtung 10 auf einem Reaktionsgefäß 50 dient, was später unter Bezugnahme auf die Figuren 2 und 3 noch beschrieben wird. Wie die Federelemente 22 können auch die Federelemente 24 mit konvexen bzw. konkaven Krümmungen 24b bzw. 24c ausgebildet sein.

Die hier dargestellten Formen der Federelemente 22 und 24 mit gebogenen bzw. gekrümmten Abschnitten 22b, 22c, 24b, 24c sind bevorzugte Ausführungsbeispiele. Alternativ können die Federelemente auch im Wesentlichen geradlinig ausgeführt sein, wobei sie in einem solchen Fall geneigt zur Innenseite des Hauptkörpers 14 verlaufen.

Die Federelemente 22, 24 sind in dieser Ausführungsform entlang dem Umfang des Hauptkörpers 14 bzw. der Abdeckvorrichtung 10 verteilt angeordnet. Die Federelemente 22 sind dabei regelmäßig verteilt entlang der beiden Querseiten 26 angeordnet. Die Federelemente 24 sind regelmäßig verteilt entlang der beiden Längsseiten 28 angeordnet.

Die Abdeckvorrichtung 10 umfasst ferner Kopplungsmittel 38, die hier als Kopplungsöffnungen 38 ausgebildet sind. Die Kopplungsmittel sind bevorzugt am oberen Rand 16a vorgesehen. In die Kopplungsöffnungen 38 können korrespondierende Nocken einer hier nicht dargestellten Greifvorrichtung eingeführt werden, um eine Abdeckvorrichtung 10 zu ergreifen, zu transportieren und an einer gewünschten Position wieder abzusetzen.

Die Abdeckvorrichtung 10 ist dazu vorgesehen, ein Reaktionsgefäß 50 (Fig. 2), wie etwa eine PCR-Platte, eine Mikrotiterplatte oder andere derartige Reaktionsgefäße abzudecken und für eine gewünschte Zeit während einem oder mehrere Reaktionsschritte eines Analyseverfahrens dicht zu verschließen. Bevorzugt handelt es sich bei den abzudeckenden Reaktionsgefäßen 50 um in der Regel rechteckige Mikrotiterplatten. Diese sind üblicherweise aus aus Kunststoff, wie etwa Polystyrole, Polyvinylchlorid, für gewisse Anwendungen auch aus Glas hergestellt. In den Reaktionsgefäßen 50 sind voneinander getrennte bzw. isolierte Vertiefungen 52 angeordnet, die auch als Näpfchen, Kavitäten oder in Englisch als "wells" bezeichnet werden. Die Vertiefungen 52 sind üblicherweise in Reihen und Spalten angeordnet. Die Normung solcher Mikrotiterplatten basiert auf ANSI Standards (ANSI/SBS 1-2004, ANSI/SBS 2-2004, ANSI/SBS 3-2004, ANSI/SBS 4-2004). Die genauen Dimensionen solcher genormter Mikrotiterplatten (Länge×Breite×Höhe) betragen gemäß ANSI-Standards 127,76 mm × 85,48 mm × 14,35 mm. Bei diesen Mikrotiterplatten bzw. Reaktionsgefäßen 50 gibt eine Vielzahl an Ausgestaltungen mit unterschiedlicher Anzahl an Vertiefungen 52, wobei diese verschiedenen Mikrotiterplatten in der Regel die gleiche Grundfläche aufweisen, aber ggf. andere Höhen.

Der Hauptkörper 14 ist bevorzugt im Wesentliches eben und bevorzugt steif ausgebildet und aus einem Kunststoff hergestellt. Das Dichtmittel 36 ist ebenfalls aus einem Kunststoff hergestellt und fest mit der Innenseite 34 des Hauptkörpers 14 verbunden. Hinsichtlich der möglichen Materialien und Dicken von Hauptkörper 14 und Dichtmittel 36 wird auf die Beschreibungseinleitung und die Ansprüche verwiesen.

Fig. 2 zeigt in Aufsichtsdarstellung und einer zugehörigen Schnittdarstellung entsprechend der Schnittlinie C-C und seitlichen Aufrissdarstellungen ein Reaktionsgefäß 50, das beispielhaft 96 Vertiefungen 52 umfasst und auf dem eine Abdeckvorrichtung 10 entsprechend der ersten Ausführungsform (Fig. 1) angeordnet ist. Die Vertiefungen 52 bzw. weitere Komponenten des Reaktionsgefäßes 50 sind in der Aufsichtsdarstellung gestrichelt dargestellt, da sie unter der Abdeckvorrichtung 10 angeordnet sind. Es wird darauf hingewiesen, dass in der Fig. 2 auch Bezugszeichen enthalten sind, die unter Bezugnahme auf die Fig. 1 beschrieben worden sind, um eine einfache Referenzierung von gleichen Bauteilen zu ermöglichen ohne wiederholende Beschreibung.

Aus der Schnittdarstellung entsprechend der Schnittlinie C-C ist ersichtlich, dass die Abdeckvorrichtung 10 mittels ihrer Federelemente 22, 24, insbesondere mittels deren Auflagebereichen 22d, 24d (Fig. 1, I und II) auf einer Oberseite 54 des Reaktionsgefäßes 50 aufliegt. Im hier dargestellten Zustand mit entspannten Federelementen 22, 24 ist ein Abstand bzw. Zwischenraum 56 zwischen der Oberseite 54 des Reaktionsgefäßes 50 und dem Dichtmittel 36 gebildet, so dass die Vertiefungen 52 (noch) nicht dicht verschlossen sind.

Dieser Zustand mit entspannten Federelementen 22, 24 ist in etwas vergrößerter Schnittdarstellung entsprechend der Schnittlinie C-C der Fig. 2 auch in der Fig. 3a und einer zugehörigen Schnittvergrößerung III (beispielhaft für die Federelemente 22) dargestellt. In Fig. 3b ist ist die Abdeckvorrichtung 10 in einer sogenannten Dichtposition dargestellt. Dabei wird die Abdeckvorrichtung an ihrer Außenseite 12 mit einem gleichmäßigen Druck P beaufschlagt. Durch diesen Druck P wird die Abdeckvorrichtung 10 relativ zum Reaktionsgefäß 50 bewegt, insbesondere wird sie zur Oberseite 54 des Reaktionsgefäßes hin bewegt bis die Abdeckvorrichtung 10 mit ihrem Dichtmittel 36 auf der Oberseite 54, insbesondere auf zur Oberseite 54 zugehörigen Öffnungsrändern 54a der Vertiefungen 52 aufliegt, so dass die Vertiefungen 52 dicht verschlossen sind. Bei dieser Bewegung der Abdeckvorrichtung 10 in Richtung der Oberseite 54 des Reaktionsgefäßes 50 werden die Federelemente 22, 24 verformt bzw. vorgespannt. Dabei werden die Federelemente in Richtung der Federöffnungen 30 bzw. 32 ausgelenkt und zumindest teilweise in diesen Federöffnungen 30, 32 aufgenommen. Der Druck P wird beispielsweise durch einen Gerätedeckel eines Thermocyclers oder eines Thermoblocks erzeugt, in dem das Reaktionsgefäß 50 und die Abdeckvorrichtung 10 aufgenommen sind. Das dichte Verschließen der Vertiefungen 52 dient dabei insbesondere dazu, dass die in den Vertiefungen 52 aufgenommene Probenflüssigkeit nicht entweicht bzw. nicht verdunstet bei einer in einem Analyseverfahren notwendigen Temperaturänderung (Erwärmung).

Lässt der aufgebrachte Druck P nach, entspannen sich die Federelemente 22, 24 wieder, wodurch die Abdeckvorrichtung 10, insbesondere der Hauptkörper 14 und das daran befestigte Dichtmittel 36 gleichzeitig und vollständig von der Oberseite 54 bzw. den Randbereichen 54a abgehoben wird, so dass wieder der Zustand entsprechend der Fig. 3a (Vergrößerung III) erreicht wird. Die von den Federelementen 22, 24 ausgeübte Vorspannkraft und die ebene bzw. steife Ausführung des Hauptkörpers ermöglichen dabei das Überwinden von gegebenenfalls in den Vertiefungen 52 entstandenem Unterdruck, der ein Anhaften des Dichtmittels 36 auf der Oberseite 54 hervorrufen kann. Die Abdeckvorrichtung 10 liegt somit wieder in einem losen Zustand mit entspannten Federelementen 22, 24 auf dem Reaktionsgefäß auf, ohne dass sie noch am Reaktionsgefäß anhaftet, und kann in einfacher und bevorzugt automatisierte Weise vom Reaktionsgefäß abgehoben und entfernt werden.

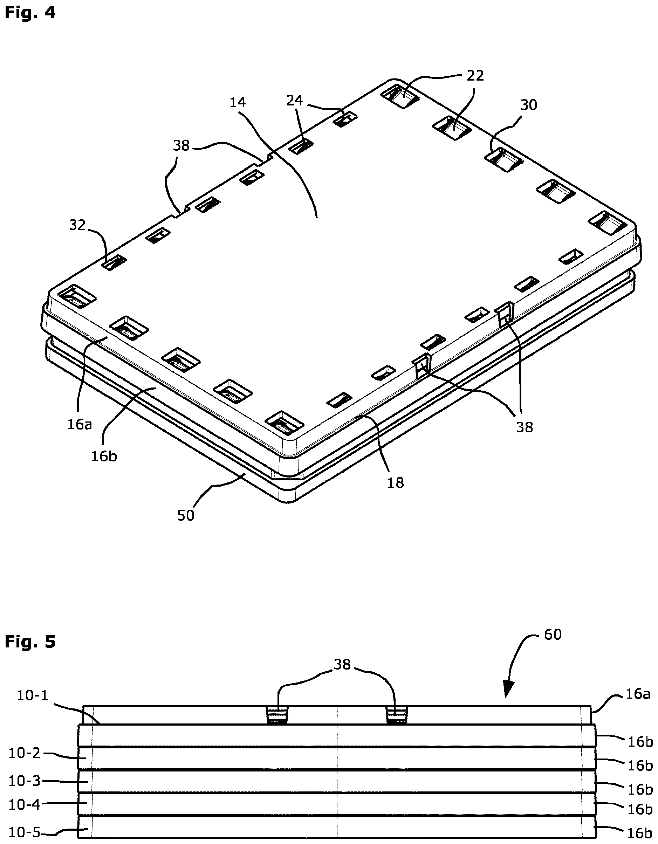

Fig. 4 zeigt in perspektivischer Darstellung die Abdeckvorrichtung 10 mit dem Reaktionsgefäß 50. Aus dieser Darstellung sind insbesondere die Kopplungsöffnungen 38 ersichtlich, an denen die Abdeckvorrichtung 10 mittels einer Greifvorrichtung ergriffen und vom Reaktionsgefäß 50 angehoben und entfernt werden kann. Ferner ist aus dieser Darstellung, insbesondere in Kombination mit der vergrößerten Schnittdarstellung I der Fig. 1 der stufenartige Vorsprung 18 des umlaufenden Randabschnitts 16 ersichtlich. Dieser stufenartige Vorsprung bietet den Vorteil, dass mehrere Abdeckvorrichtungen 10-1, bis 10-5 als Stapel 60 aufeinander angeordnet werden können, wie dies aus der Fig. 5 ersichtlich ist. Der stufenartige Vorsprung 18 ist so dimensioniert, dass eine obere Abdeckvorrichtung 10-1 mit ihrem unteren Rand 16b, auf dem stufenartigen Vorsprung 18 einer direkt unterhalb liegenden Abdeckvorrichtung 10-2 abgestützt werden kann. Da die Ränder 16a und 16b einer Abdeckvorrichtung 10-1 bis 10-5 etwa je die Hälfte der gesamten Höhe des Randabschnitts 16 ausmachen, kann die Packungsdichte eines Stapels 60 von Abdeckvorrichtungen 10 in etwa verdoppelt werden. Das Hinzufügen von Abdeckvorrichtungen 10-1 bis 10-5 zu einem Stapel 60 und auch das Abheben einer obersten Abdeckvorrichtung 10-1 von dem Stapel 60 wird ferner unterstützt durch die bezogen auf eine Vertikale leicht geneigten Ränder 16a und 16b. Anders ausgedrückt schließen die Ränder 16a und 16b mit der Ebene des Hauptkörpers einen Winkel ein der etwas größer ist als 90°, vorzugsweise aber kleiner als 100°. Entsprechend ist auch der stufenartige Vorsprung 18 bezüglich der Ränder 16a, 16b im Wesentlichen orthogonal ausgerichtet. Bevorzugt liegt der stufenartige Vorsprung 18 in einer zum Hauptkörper 14 parallelen Ebene, kann aber auch leicht geneigt zu einer solchen Ebene verlaufen. Da die Abdeckvorrichtungen 10-1 bis 10-5 im Stapel 16 auf ihren jeweiligen unteren Rändern 16b stehen, werden die Federelemente 22, 24 im gestapelten Zustand nicht verformt und können in ihrer entspannten Lage gelagert werden, so dass das Risiko einer plastischen Verformung der Federelemente 22, 24 aufgrund lang dauernd einwirkender Kräfte ausgeschlossen werden kann.

Ein Fig. 5 dargestellter Stapel 60 kann selbstverständlich mehr oder weniger als die fünf dargestellten Abdeckvorrichtungen 10-1 bis 10-5 umfassen. Bei der Verwendung von solchen Abdeckvorrichtungen 10, können je nach zur Verfügung stehendem Raum in einer automatisierten Analysevorrichtung auch mehrere Stapel zum Einsatz kommen, von denen dann kontinuierlich Abdeckvorrichtungen 10 entnommen werden zum Abdecken und Abdichten von Reaktionsgefäßen 50. Die Abdeckvorrichtungen 10 kommen bevorzugt als Einweg-Verbrauchsmaterial zum Einsatz und werden nach dem Abdecken/Abdichten eines Reaktionsgefäßes entsorgt, um aufwändige Reinigungsarbeiten zu vermeiden und um das Risiko von Kontamination zu verringern.

Unter Verwendung eines solchen Stapels 60 kann ein Verfahren zum Abdecken von Reaktionsgefäßen 50 durchgeführt werden, das folgende Schritte umfassen kann:
a) Bereitstellen eines Stapels 60 mit mehreren Abdeckvorrichtungen 10-1 bis 10-5
b) Bereitstellen von wenigstens einem abzudeckenden Reaktionsgefäß 50, insbesondere einer PCR-Platte oder einer Mikrotiterplatte;
c) Ergreifen der obersten Abdeckvorrichtung 10-1 des Stapels 60 mittels einer hier nicht dargestellten Greifvorrichtung;
d) Bewegen der ergriffenen Abdeckvorrichtung 10-1 zu dem bzw. zu einem abzudeckenden Reaktionsgefäß 50;
   Absetzen der ergriffenen Abdeckvorrichtung 10-1 auf dem gewünschten Reaktionsgefäß 50;
f) Druck ausüben auf die Abdeckvorrichtung 10-1 auf dem Reaktionsgefäß 50 unter Verformung und Vorspannung der Federelemente 22, 24 der Abdeckvorrichtung 10-1, um die Vertiefungen 52 im Reaktionsgefäß 50 mittels des Dichtmittels 36 dicht zu verschließen;
g) Durchführen von für ein gewünschtes Analyseverfahren erforderlichen Schritten mit verschlossenem Reaktionsgefäß 50, wie etwa Temperatur erhöhen oder/und senken;
h) Freigabe der Abdeckvorrichtung 10-1 durch Nachlassen des aufgebrachten Drucks unter Rückverformung und Entspannung der Federelemente 22, 24, um das Dichtmittel 36 vollständig von dem Reaktionsgefäß 50 zu lösen;
i) Ergreifen der gebrauchten Abdeckvorrichtung 10-1 mittels einer Greifvorrichtung und Entsorgen der Abdeckvorrichtung 10-1.

Bei einem solchen Verfahren können die Schritte a) bis e) wiederholt ausgeführt werden, so dass Nacheinander mehrere Reaktionsgefäße mit weiteren jeweiligen Abdeckvorrichtungen 10-2 bis 10-5 vorzugsweise aus dem Stapel 60 abgedeckt werden. Die Schritte f) bis h) können auch gleichzeitig für mehrere oder alle abgedeckten Reaktionsgefäße durchgeführt werden. Schließlich kann der Schritt i) wiederholt durchgeführt werden bis alle verwendeten Abdeckvorrichtungen 10-2 bis 10-5 von den jeweiligen Reaktionsgefäßen entfernt sind.

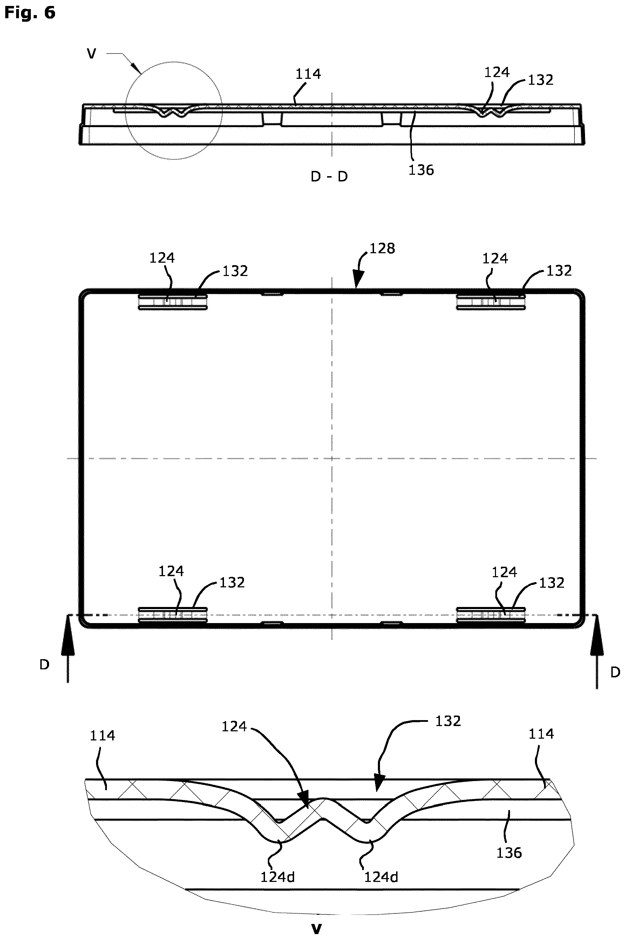

Fig. 6 zeigt eine zweite Ausführungsform einer Abdeckvorrichtung 110 mit Federelementen 124, die am Hauptkörper 114 entlang der Längsseiten 128 befestigt sind. Die Federelemente 124 sind in der Art von wellenförmigen Stegen ausgeführt und weisen zwei Auflagebereiche 124d, mit denen sie auf der Oberseite 54 eines Reaktionsgefäßes 50 (Fig. 2 und 3) aufliegen können. Die Federelemente 124 sind beidseitig mit dem Hauptkörper 114 verbunden und weisen kein freies Ende auf. Durch die wellenartige Formgebung können sich diese Federelemente 124 jedoch ebenfalls verformen und vorgespannt werden, wenn Druck auf den Hauptkörper 114 ausgeübt wird. Die Federelemente 124 bewegen sich dann wenigstens teilweise in korrespondierende Federöffnungen 132 im Hauptkörper 114.

Selbstverständlich ist auch in dieser Ausführungsform ein Dichtmittel 136 mit dem Hauptkörper 114 verbunden. Im Übrigen ist die Funktionsweise und der Einsatz der Abdeckvorrichtung 110 gleich wie bei der Abdeckvorrichtung 10 der Fig. 1 bis 4. Auch die Abdeckungsvorrichtung 110 weist eine entsprechende Formgebung (Randabschnitt) auf wie die erste Ausführungsform, damit mehrere solche Abdeckungsvorrichtungen 110 als Stapel aufeinander angeordnet werden können.

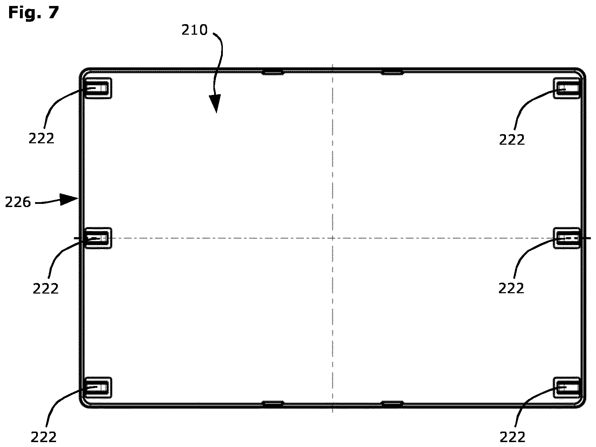

Schließlich zeigt die Fig. 7 eine dritte Ausführungsform einer Abdeckvorrichtung 210 mit einer geringeren Anzahl Federelementen 222 entlang der Querseiten 226 der Abdeckvorrichtung. Die Federelemente 222 sind verglichen mit denjenigen der Fig. 1 ehe länglich ausgebildet, weisen aber vom Grundsatz her die gleiche Funktion und Wirkungsweise auf. Auch diese Ausführungsform ist stapelbar und weist einen zur ersten Ausführungsform gleichen oder ähnlichen Randabschnitt auf.

Für alle Ausführungsbeispiel beträgt die Dicke des Hauptkörpers 14 etwa 0,5 bis 3,0 mm , insbesondere etwa 1,0 bis 2,0 mm, und die Dicke des Dichtmittels 36 etwa 0,5 bis 3,0 mm, insbesondere etwa 1,0 bis 2,0 mm. Aus der Zusammenschau der verschiedenen Ausführungsformen ist ferner klar ersichtlich, dass die Ausgestaltung der Federelemente und deren Anordnung im Hauptkörper bzw. deren Anzahl veränderlich und wählbar ist. Es hat sich gezeigt, dass bei Abdeckungsvorrichtungen, die eine im Wesentlichen rechteckige Grundform aufweisen, eine gerade Anzahl von Federelementen vorteilhaft ist, um diese symmetrisch anordnen zu können und um eine gleichmäßige Wirkung der Federelemente auf den Hauptkörper bzw. die Abdeckvorrichtung zu erreichen, insbesondere wenn die Federelemente von ihrem vorgespannten Zustand in den entspannten Zustand bewegt werden.

Die hier vorgestellten Abdeckungsvorrichtungen kommen bevorzugt in automatisiert arbeitenden Analyse- bzw. Dosiervorrichtungen, wie etwa Pipettierroboter, zum Einsatz. Solche Analyse- bzw. Dosiervorrichtungen verfügen üblicherweise über entsprechende Antriebe und Steuerungseinrichtungen, um die oben beschriebenen Verfahrensschritte unter Verwendung von oben beschriebenen Abdeckvorrichtungen durchzuführen, inklusive das Ergreifen und Transportieren von solchen Abdeckvorrichtungen. Dabei kann für die automatisierte Handhabung vorzugsweise die gleiche Greifvorrichtung zum Einsatz kommen, die auch für das Ergreifen und den Transport von Reaktionsgefäßen verwendet wird.

## Patentansprüche

1. Abdeckvorrichtung (10), für die gleichzeitige Abdeckung von in einem Reaktionsgefäß (50) ausgebildeten Probenbehältern (52), insbesondere von Vertiefungen (52) in PCR-Platten oder Mikrotiterplatten, wobei die Abdeckvorrichtung dazu eingerichtet ist, die Probenbehälter (52) des Reaktionsgefäßes (50) gleichzeitig für eine gewünschte Zeit dicht zu verschließen, umfassend:
einen ebenen Hauptkörper (14) mit einer Innenseite (34) und einer Außenseite (12),
wenigstens ein am Hauptkörper (14) angeordnetes und mit diesem verbundenes, flächiges Dichtmittel (36), wobei das wenigstens eine Dichtmittel (36) an der Innenseite (34) des Hauptkörpers (14) angeordnet ist, und wobei der Hauptkörper (14) zwei parallele Längsseiten (28) und zwei parallele Querseiten (26) aufweist, einen dem Umfang des Hauptkörpers entlang verlaufenden Randabschnitt (16), der sich von der Außenseite (12) in Richtung der Innenseite (34) und über diese hinaus erstreckt, **gekennzeichnet durch** mehrere am Hauptkörper (14) angeordnete Federelemente (22, 24; 124; 222), die entlang der Längsseiten (28) oder/und entlang der Querseiten (26) angeordnet sind, wobei die Federelemente (22, 24; 124; 222) derart ausgeführt sind, dass die Abdeckvorrichtung (10) mittels der in einem entspannten Zustand befindlichen Federelemente (22, 24; 124; 222) auf einer der Abdeckvorrichtung (10) zugewandten Oberfläche (54) eines abzudeckenden Reaktionsgefäßes (50) unter Ausbildung eines Zwischenraums (56) zwischen dem Dichtmittel (36) und der Oberfläche 54 abstützbar ist, wobei die Federelemente (22, 24; 124; 222) derart ausgeführt sind, dass in ihrem gespannten Zustand das Dichtmittel (36) auf der Oberfläche (54) des abgedeckten Reaktionsgefäßes (50) aufliegt, um die im Reaktionsgefäß angeordneten Probenbehälter (52) dicht zu verschließen.

2. Abdeckvorrichtung nach Anspruch 1, wobei die Federelemente mit wenigstens einem Ende am Hauptkörper oder/und am Randabschnitt befestigt sind, vorzugsweise einstückig mit dem Hauptkörper oder/und dem Randabschnitt ausgebildet sind, wobei vorzugsweise die Federelemente (22, 24; 124; 222) wenigstens abschnittsweise gebogen ausgeführt sind mit wenigstens einer bezogen auf die Innenseite (34) des Hauptkörpers (14) konvexen oder/und konkaven Krümmung (22b, 22c, 24b).

3. Abdeckvorrichtung nach Anspruch 1 oder 2, wobei die Federelemente (22, 24; 124; 222) als einseitig oder zweiseitig am Hauptkörper (14) oder/und am Randabschnitt (16) befestigte blattfederartige Stege ausgebildet sind.

4. Abdeckvorrichtung nach Anspruch 3, wobei der blattfederartige Steg (22, 24) einseitig am Randabschnitt (16) oder am Hauptkörper (14) befestigt ist mit einem beweglichen freien Ende (22a, 24a), oder wobei der blattfederartige Steg (124) zweiseitig am Hauptkörper (114) oder Dichtmittel befestigt ist und der Steg (124) zwischen den beiden Befestigungsbereichen mehrere wellenartige Krümmungen (124d) aufweist.

5. Abdeckvorrichtung nach Anspruch 3 oder 4, wobei der Hauptkörper (14) den Federelementen (22, 24; 124; 222) zugeordnete Federöffnungen (30, 32; 132) umfasst, die derart ausgebildet sind, dass sie im gespannten Zustand der Federelemente (22, 24; 124; 222) diese wenigstens teilweise aufnehmen, insbesondere deren bewegliche freie Enden (22a, 24a).

6. Abdeckvorrichtung nach einem der vorhergehenden Ansprüche, wobei sie eine gerade Anzahl von Federelementen (22, 24; 124; 222) umfasst, wobei vorzugsweise die Federelemente (22, 24; 124; 222) entlang des Umfangs des Hauptkörpers (14) verteilt angeordnet sind.

7. Abdeckvorrichtung nach einem der vorhergehenden Ansprüche, wobei der Randabschnitt (16) einen sich am Hauptkörper (14) anschließenden oberen Rand (16a) und einen mit dem oberen Rand (16a) verbundenen unteren Rand (16b) aufweist, wobei ein entlang des oberen Randes (16a) gemessener Umfang kleiner ist als der Umfang entlang des unteren Randes (16b), wobei vorzugsweise der obere Rand (16a) und der untere Rand (16b) durch einen stufenartigen umlaufenden Vorsprung (18) miteinander verbunden sind, der zum oberen und zum unteren Rand (16a, 16b) geneigt verläuft, vorzugsweise im Wesentlichen orthogonal verläuft.

8. Abdeckvorrichtung nach Anspruch 7, wobei der untere Rand (16b) und der stufenartige Vorsprung (18) derart dimensioniert sind, dass die Abdeckvorrichtung (10-1) auf einer weiteren gleichartigen Abdeckvorrichtung (10-2) stapelbar ist, wobei im gestapelten Zustand die Abdeckvorrichtung (10-1) mit ihrem unteren Rand (16b) auf dem stufenartigen Vorsprung (18) der darunter angeordneten weiteren Abdeckvorrichtung (10-2) aufliegt.

9. Abdeckvorrichtung nach einem der vorhergehenden Ansprüche, wobei im Randabschnitt (16), vorzugsweise im oberen Rand (16a), Kopplungsmittel (38) vorgesehen sind, um die Abdeckvorrichtung (10) mittels einer Greifvorrichtung lösbar aufzunehmen und zu transportieren, wobei die Kopplungsmittel (38) vorzugsweise als Öffnungen im Randabschnitt (16) ausgebildet sind, in die korrespondierende Nocken einer Greifvorrichtung einführbar sind.

10. Abdeckvorrichtung nach einem der vorhergehenden Ansprüche, wobei die Federelemente (22, 24; 124; 222) entlang der Längsseiten (28) oder/und entlang der Querseiten (26) regelmäßig verteilt sind.

11. Abdeckvorrichtung, nach einem der vorhergehenden Ansprüche, wobei der Hauptkörper (14), der Randabschnitt (16) und die Federelemente (22, 24; 124; 222) aus einem Kunststoff hergestellt sind, vorzugsweise aus einem spritzgussfähigen Kunststoff, wie etwa Polycarbonat (PC), Polymerblends (ASB-PC), Polyamid (PA) oder Polybutylenterephtalat (PBT), wobei der Hauptkörper (14), der Randabschnitt (16) und die Federelemente (22, 24; 124; 222) vorzugsweise einstückig ausgebildet sind, und wobei vorzugsweise das Dichtmittel (36) aus einem thermoplastischen Elastomer (TPE) oder einem silikonhaltigen Elastomer hergestellt ist.

12. Abdeckvorrichtung nach einem der vorhergehenden Ansprüche, wobei die Dicke des Hauptkörpers (14) zwischen seiner Außenseite (12) und seiner Innenseite (34) 0,5 bis 3,0 mm beträgt, insbesondere 1,0 bis 2,0 mm, und wobei vorzugsweise die Dicke des Dichtmittels (36) 0,5 bis 3,0 mm beträgt, insbesondere 1,0 bis 2,0 mm.

13. Reaktionsgefäß (50), insbesondere PCR-Platte oder Mikrotiterplatte, umfassend mehrere Vertiefungen (52) bzw. Kavitäten als Probenbehälter, wobei das Reaktionsgefäß (50) mit einer Abdeckvorrichtung (10) nach einem der vorhergehenden Ansprüche abgedeckt ist, wobei vorzugsweise das Reaktionsgefäß (50) nach den AN-SI-Standards ANSI/SLAS 1-2004 bis ANSI/SLAS 4-2004 ausgeführt ist.

14. Verfahren zum Abdecken von Reaktionsgefäßen (50) umfassend die Schritte:
a) Bereitstellen eines Stapels (60) mit mehreren Abdeckvorrichtungen (10; 10-1,..., 10-5) nach einem der Ansprüche 1 bis 12;
b) Bereitstellen von wenigstens einem abzudeckenden Reaktionsgefäß (50), insbesondere einer PCR-Platte oder einer Mikrotiterplatte;
c) Ergreifen einer obersten Abdeckvorrichtung (10-1) des Stapels (60) mittels einer Greifvorrichtung;
d) Bewegen der ergriffenen Abdeckvorrichtung (10-1) zu dem bzw. zu einem abzudeckenden Reaktionsgefäß (50);
e) Absetzen der ergriffenen Abdeckvorrichtung (10-1) auf dem Reaktionsgefäß (50);
f) Druck ausüben auf die Abdeckvorrichtung (10-1) auf dem Reaktionsgefäß (50) unter Verformung und Vorspannung der Federelemente (22, 24; 124; 222) der Abdeckvorrichtung (10-1), um Vertiefungen (52) im Reaktionsgefäß (50) mittels des Dichtmittels (36) dicht zu verschließen;
g) Durchführen von für ein gewünschtes Analyseverfahren erforderlichen Schritten mit verschlossenem Reaktionsgefäß (50), wie etwa Temperatur erhöhen oder/und senken;
h) Freigabe der Abdeckvorrichtung (10-1) durch Nachlassen des aufgebrachten Drucks unter Rückverformung und Entspannung der Federelemente (22, 24; 124;222), um das Dichtmittel (36) vollständig von dem Reaktionsgefäß (50) zu lösen;
i) Ergreifen der gebrauchten Abdeckvorrichtung (10-1) mittels einer Greifvorrichtung und Entsorgen der Abdeckvorrichtung (10-1).

15. Verfahren nach Anspruch 14 wobei die Schritte a) bis e) wiederholt ausgeführt werden, so dass Nacheinander mehrere Reaktionsgefäße mit jeweiligen Abdeckvorrichtungen (10-1,..., 10-5) abgedeckt werden, wobei die Schritte f) bis h) vorzugsweise gleichzeitig für alle abgedeckten Reaktionsgefäße durchgeführt werden und wobei vorzugsweise der Schritt i) wiederholt durchgeführt wird bis alle verwendeten Abdeckvorrichtungen (10-1,..., 10-5) von den jeweiligen Reaktionsgefäßen entfernt sind, wobei vorzugsweise die Schritte durch eine automatisierte Dosiervorrichtung, insbesondere einen Pipettierroboter durchgeführt werden.

16. Dosiervorrichtung, insbesondere Pipettierroboter, umfassend:
eine Auflageplatte, auf welcher ein Stapel (60) aus nicht gebrauchten Abdeckvorrichtungen (10-1,..., 10-5) gemäß einem der Ansprüche 1 bis 12 positioniert ist und auf welcher wenigstens ein Reaktionsgefäß (50) positioniert ist, und
eine relativ zur Aufnahmeplatte in drei Hauptrichtung bewegliche Greifvorrichtung, die dazu eingerichtet ist, Abdeckvorrichtungen (10-1,..., 10-5) vom Stapel zu ergreifen und auf dem Reaktionsgefäß (50) abzusetzen, sowie eine gebrauchte Abdeckvorrichtung (10-1,..., 10-5) von einem Reaktionsgefäß (50) zu entfernen und zu entsorgen.

## Claims

1. A covering device (10) for covering simultaneously sample containers (52) formed in a reaction vessel (50), in particular of recesses (52) in PCR plates or microtiter plates, wherein the covering device is configured to tightly seal the sample containers (52) of the reaction vessel (50) simultaneously for a desired time, the covering device comprising:
a flat main body (14) having an inside (34) and an outside (12),
at least one planar sealing element (36) arranged on the main body (14) and connected to the main body (14), wherein the at least one sealing element (36) is arranged on the inside (34) of the main body (14), and wherein the main body (14) comprises two parallel longitudinal sides (28) and two parallel transversal sides (26),
an edge segment (16), which runs along the periphery of the main body and extends from the outside (12) in the direction of the inside (34) and beyond the inside,
**characterized by**
a plurality of spring elements (22, 24; 124; 222) arranged on the main body (14), which are arranged along the longitudinal sides (28) and/or along the transversal sides (26), wherein the spring elements (22, 24; 124; 222) are formed in such a way that the covering device (10) is supportable by means of the spring elements (22, 24; 124; 222), when the spring elements are in a relaxed state, on a surface (54) of a reaction vessel (50) to be covered facing the covering device (10) under formation of an intermediate space (56) between the sealing element (36) and the surface (54), wherein
the spring elements (22, 24; 124; 222) are formed such that in a strained state the sealing element (36) rests on the surface (54) of the covered reaction vessel (50), in order to tightly seal the sample containers (52) that are arranged in the sampling vessel.

2. The covering device according to claim 1, wherein the spring elements are fixed with at least one end on the main body and/or the edge segment, and are preferably formed integrally with the main body and/or the edge segment, wherein preferably the spring elements (22, 24; 124; 222) are formed bent at least in sections with at least a convex and/or concave curvature (22b, 22c, 24b) with respect to the inside (34) of the main body (14).

3. The covering device according claim 1 or 2, wherein the spring elements (22, 24; 124; 222) are formed as leaf-spring-like struts that are fixed single-sided or double-sided to the main body (14) and/or the edge segment (16).

4. The covering device according to claim 3, wherein the leaf-spring-like strut (22, 24) is fixed single-sided to the edge segment (16) or the main body (14) and has a movable free end (22a, 24a) or wherein the leaf-spring-like strut (124) is fixed double-sided on the main body (114) or the sealing element and the strut (124) comprises several wave-like curvatures (124d) between the two fixing regions.

5. The covering device according to claim 3 or 4, wherein the main body (14) comprises spring openings (30, 32; 132) assigned to the spring elements (22, 24; 124; 222), which are formed such that in the strained state the spring elements (22, 24; 124; 222) are contained at least partially in the spring openings, in particular their movable free ends (22a, 24a).

6. The covering device according to one of the preceding claims, wherein the covering device comprises an even number of spring elements (22, 24; 124; 222), wherein preferably the spring elements (22, 24; 124; 222) are arranged in a distributed manner along the periphery of the main body (14).

7. The covering device according to one of the preceding claims, wherein the edge segment (16) comprises an upper rim (16a) adjacent to the main body (14) and a lower rim (16b) connected to the upper rim (16a), wherein a circumference measured along the upper rim (16a) is smaller than the circumference along the lower rim (16b), wherein preferably the upper rim (16a) and the lower rim (16b) are connected with each other by means of a step-like circumferential protrusion (18), which is inclined, preferably substantial orthogonal, with respect to the upper and the lower rim (16a, 16b).

8. The covering device according to claim 7, wherein the lower rim (16b) and the step-like protrusion (18) are dimensioned such that the covering device (10-1) is stackable on a further similar covering device (10-2), wherein in the stacked state the covering device (10-1) rests with its lower rim (16b) on the step-like protrusion (18) of the further covering device (10-2) arranged thereunder.

9. The covering device according to one of the preceding claims, wherein in the edge segment (16), preferably in its upper rim (16a), coupling means (38) are provided in order to releasable take up and transport the covering device (10) by means of a gripping device, wherein the coupling means (38) are preferably formed as openings in the edge segment (16), into which corresponding lobes of a gripping device can be inserted.

10. The covering device according to one of the preceding claims, wherein the spring elements (22, 24; 124; 222) are arranged in a regularly distributed manner along the longitudinal sides (28) and/or along the transversal sides (26).

11. The covering device according to one of the preceding claims, wherein the main body (14), the edge segment (16), and the spring elements (22, 24; 124; 222) are formed from a plastic material, preferably from an injection moldable plastic material, such as polycarbonate (PC), polymer blends (ASB-PC), polyamide (PA) or polybutylenterephtalat (PBT), wherein the main body (14), the edge segment (16), and the spring elements (22, 24; 124; 222) are preferably formed integrally, and wherein the sealing element (36) is preferably formed from a thermoplastic elastomer (TPE) or a silicone containing elastomer.

12. The covering device according to one of the preceding claims, wherein the width of the main body (14) between its outside (12) and its inside (34) is 0.5 to 3.0 mm, in particular 1.0 to 2.0 mm, and wherein preferably the width of the sealing element (36) is 0.5 to 3.0 mm, in particular about 1.0 to 2.0 mm.

13. A reaction vessel (50), in particular a PCR plate or a microtiter plate, comprising several recesses (52) or cavities as sample containers, wherein the reaction vessel (50) is covered with a covering device (10) according to one of the preceding claims, wherein the reaction vessel (50) is preferably formed according to the ANSI standards ANSI/SLAS 1-2004 to ANSI/SLAS 4-2004.

14. A method for covering reaction vessels (50) comprising the steps:
a) providing a stack (60) comprising several covering devices (10; 10-1,..., 10-5) according to one of claims 1 to 12;
b) providing at least one reaction vessel (50) to be covered, in particular a PCR plate or a microtiter plate;
c) taking up an uppermost covering device (10-1) of the stack (60) by means of a gripping device;
d) moving the taken covering device (10-1) to the or to a reaction vessel (50) to be covered;
e) placing the taken covering device (10-1) onto the reaction vessel (50);
f) applying pressure onto the covering device (10-1) on the reaction vessel (50) under deformation and pre-tension of the spring elements (22, 24; 124; 222) of the covering device (10-1), in order to tightly seal recesses (52) in the reaction vessel (50) by means of the sealing element (36);
g) carrying out steps necessary for a desired analysis method with closed reaction vessel (50), such as increasing and/or decreasing a temperature;
h) releasing the covering device (10-1) by reducing the applied pressure under recovery and relaxation of the spring elements (22, 24; 124; 222) to release the sealing element (36) completely from the reaction vessel (50);
i) taking up the used covering device (10-1) by means of a gripping device and disposing of the covering device (10-1).

15. The method according to claim 14, wherein the steps a) to e) are carried out repeatedly such that several reaction vessels are covered consecutively with corresponding covering devices (10-1,..., 10-5), wherein the steps f) to h) are preferably carried out simultaneously for all covered reaction vessels and wherein preferably the step i) is carried out repeatedly until all used covering device (10-1,..., 10-5) are removed from the respective reaction vessels, wherein preferably the steps are carried out by an automated dosing device, in particular by a pipetting robot.

16. A dosing device, in particular a pipetting robot, comprising:
a support plate on which a stack (60) of non-used covering devices (10-1, ..., 10-5) according to one of claims 1 to 12 is positioned and on which at least one reaction vessel (50) is positioned, and
a gripping device that is movable relative to the support plate in three main directions and configured to take up covering devices (10-1, ..., 10-5) from the stack, to place the covering devices on the reaction vessel (50), and to remove and dispose a used covering devices (10-1,..., 10-5) from a reaction vessel (50).

## Revendications

1. Dispositif de recouvrement (10) pour le recouvrement simultané de récipients d'échantillon (52) formés dans un récipient de réaction (50), en particulier de creux (52) dans des plaques PCR ou des plaques de microtitrage, dans lequel le dispositif de recouvrement est installé pour fermer simultanément de manière étanche les récipients d'échantillon (52) du récipient de réaction (50) pour une durée souhaitée, comprenant :
un corps principal plat (14) avec un côté intérieur (34) et un côté extérieur (12),
au moins un moyen d'étanchéité plan (36) disposé sur le corps principal (14) et relié à celui-ci, dans lequel le au moins un moyen d'étanchéité (36) est disposé sur le côté intérieur (34) du corps principal (14) et le corps principal (14) présente deux côtés longitudinaux parallèles (28) et deux côtés transversaux parallèles (26),
une section de bord (16) qui s'étend le long de la circonférence du corps principal et qui s'étend du côté extérieur (12) en direction du côté intérieur (34) et au-delà de celui-ci, **caractérisé par** plusieurs éléments à ressort (22, 24 ; 124 ; 222) disposés sur le corps principal (14), qui sont disposés le long des côtés longitudinaux (28) ou/et le long des côtés transversaux (26), dans lequel les éléments à ressort (22, 24 ; 124 ; 222) sont réalisés de telle sorte que le dispositif de recouvrement (10) puisse s'appuyer à l'aide des éléments à ressort (22, 24 ; 124 ; 222) qui se trouvent dans un état détendu sur une surface (54) d'un récipient de réaction à couvrir (50) tournée vers le dispositif de recouvrement, en formant un espace intermédiaire (56) entre le moyen d'étanchéité (36) et la surface (54), dans lequel les éléments à ressort (22, 24 ; 124 ; 222) sont réalisés de telle sorte que dans leur état tendu, le moyen d'étanchéité (36) soit posé sur la surface (54) du récipient de réaction (50) pour fermer de manière étanche les récipients d'échantillon (52) disposés dans le récipient de réaction.

2. Dispositif de recouvrement selon la revendication 1, dans lequel les éléments à ressort sont fixés avec au moins une extrémité au corps principal ou/et à la section de bord, sont formés de préférence d'un seul tenant avec le corps principal ou/et la section de bord, dans lequel les éléments à ressort (22, 24 ; 124 ; 222) sont de préférence courbes au moins par sections, avec au moins une courbure (22b, 22c, 24b) convexe ou/et concave par rapport au côté intérieur (34) du corps principal (14).

3. Dispositif de recouvrement selon la revendication 1 ou 2, dans lequel les éléments à ressort (22, 24 ; 124 ; 222) sont formés comme des languettes en forme de ressorts à lames fixées sur un côté ou sur deux côtés au corps principal (14) ou/et à la section de bord (16).

4. Dispositif de recouvrement selon la revendication 3, dans lequel la languette en forme de ressort à lames (22, 24) est fixée sur un côté à la section de bord (16) ou au corps principal (14) avec une extrémité libre mobile (22a, 24a), ou dans lequel la languette en forme de ressort à lames (124) est fixée sur deux côtés au corps principal (114) ou au moyen d'étanchéité et la languette (124) présente entre les deux zones de fixation plusieurs courbures ondulées (124d).

5. Dispositif de recouvrement selon la revendication 3 ou 4, dans lequel le corps principal (14) comprend des ouvertures pour ressort (30, 32 ; 132) associées aux éléments à ressort (22, 24 ; 124 ; 222), qui sont formées de manière à recevoir au moins partiellement les éléments à ressort (22, 24 ; 124 ; 222) à l'état tendu, en particulier les extrémités libres mobiles (22a, 24a) de ceux-ci.

6. Dispositif de recouvrement selon l'une des revendications précédentes, dans lequel il comprend un nombre pair d'éléments à ressort (22, 24 ; 124 ; 222), dans lequel les éléments à ressort (22, 24 ; 124 ; 222) sont répartis de préférence le long de la circonférence du corps principal (14).

7. Dispositif de recouvrement selon l'une des revendications précédentes, dans lequel la section de bord (16) présente un bord supérieur (16a) qui fait suite au corps principal (14) et un bord inférieur (16b) relié au bord supérieur (16a), dans lequel une circonférence mesurée le long du bord supérieur (16a) est plus petite que la circonférence le long du bord inférieur (16b), dans lequel le bord supérieur (16a) et le bord inférieur (16b) sont de préférence reliés entre eux par une saillie périphérique étagée (18) qui est inclinée vers le bord supérieur et vers le bord inférieur (16a, 16b), de préférence sensiblement orthogonale.

8. Dispositif de recouvrement selon la revendication 7, dans lequel le bord inférieur (16b) et la saillie étagée (18) sont dimensionnés de telle sorte que le dispositif de recouvrement (10-1) puisse être empilé sur un autre dispositif de recouvrement (10-2) du même type, dans lequel à l'état empilé, le dispositif de recouvrement (10-1) est posé avec son bord inférieur (16b) sur la saillie étagée (18) de l'autre dispositif de recouvrement (10-2) disposé dessous.

9. Dispositif de recouvrement selon l'une des revendications précédentes, dans lequel il est prévu dans la section de bord (16), de préférence dans le bord supérieur (16a), des moyens de couplage (38) pour recevoir de manière amovible et transporter à l'aide d'un moyen de préhension le dispositif de recouvrement (10), dans lequel les moyens de couplage (38) sont formés de préférence comme des ouvertures dans la section de bord (16) dans lesquelles les ergots correspondants d'un dispositif de préhension peuvent être introduites.

10. Dispositif de recouvrement selon l'une des revendications précédentes, dans lequel les éléments à ressort (22, 24 ; 124 ; 222) sont répartis régulièrement le long des côtés longitudinaux (28) ou/et le long des côtés transversaux (26).

11. Dispositif de recouvrement selon l'une des revendications précédentes, dans lequel le corps principal (14), la section de bord (16) et les éléments à ressort (22, 24 ; 124 ; 222) sont fabriqués dans une matière plastique, de préférence une matière plastique apte à être moulée par injection telle que du polycarbonate (PC), des mélanges de polymères (ASB-PC), du polyamide (PA) et du polybutylène térephtalate (PBT), dans lequel le corps principal (14), la section de bord (16) et les éléments à ressort (22, 24 ; 124 ; 222) sont formés de préférence d'un seul tenant, et dans lequel le moyen d'étanchéité (36) est fabriqué de préférence dans un élastomère thermoplastique (TPE) ou un élastomère contenant un silicone.

12. Dispositif de recouvrement selon l'une des revendications précédentes, dans lequel l'épaisseur du corps principal (14) entre son côté extérieur (12) et son côté intérieur (34) est de 0,5 à 3,0mm, en particulier de 1,0 à 2,0mm, et dans lequel l'épaisseur du moyen d'étanchéité (36) est de préférence de 0,5 à 3,0 mm, en particulier de 1,0 à 2,0 mm.

13. Récipient de réaction (50), en particulier plaque PCR ou plaque de microtitrage, comprenant plusieurs creux (52) ou cavités comme récipients d'échantillon, dans lequel le récipient de réaction (50) est couvert avec un dispositif de recouvrement (10) selon l'une des revendications précédentes, dans lequel le récipient de réaction (50) est de préférence réalisé selon les normes AN-SI ANSI/SLAS 1-2004 à ANSI/SLA 4-2004.

14. Procédé pour recouvrir des récipients de réaction (50), comprenant les étapes suivantes :
a) préparer un empilement (60) de plusieurs dispositifs de recouvrement (10; 10-1,..., 10-5) selon l'une des revendications 1 à 12 ;
b) préparer au moins un récipient de réaction à recouvrir (50), en particulier une plaque PCR ou une plaque de microtitrage ;
c) saisir un dispositif de recouvrement (10-1) supérieur de l'empilement (60) à l'aide d'un dispositif de préhension ;
d) déplacer le dispositif de recouvrement (10-1) saisi jusqu'au ou jusqu'à un récipient de réaction à recouvrir (50) ;
e) poser le dispositif de recouvrement (10-1) saisi sur le récipient de réaction (50) ;
f) exercer une pression sur le dispositif de recouvrement (10-1) sur le récipient de réaction (50) en déformant et précontraignant les éléments à ressort (22, 24 ; 124 ; 222) du dispositif de recouvrement (10-1) pour fermer de manière étanche les creux (52) dans le récipient de réaction à l'aide du moyen d'étanchéité (36) ;
g) réaliser des étapes nécessaires pour un procédé d'analyse souhaité, avec le récipient de réaction (50) fermé, comme par exemple augmenter ou/et abaisser la température ;
h) libérer le dispositif de recouvrement (10-1) en relâchant la pression appliquée, avec une reprise de leur forme et une détente des éléments à ressort (22, 24 ; 124 ; 222) pour détacher complètement le moyen d'étanchéité (36) du récipient de réaction (50) ;
i) saisir à l'aide d'un dispositif de préhension le dispositif de recouvrement (10-1) utilisé, et l'évacuer à l'aide d'un dispositif de préhension.

15. Procédé selon la revendication 14, dans lequel les étapes a) à e) sont réalisées à plusieurs reprises, de sorte que plusieurs récipients de réaction sont recouverts successivement avec des dispositifs de recouvrement respectifs (10-1,..., 10-5), dans lequel les étapes f) à h) sont réalisées de préférence simultanément pour tous les récipients de réaction recouverts, et dans lequel l'étape i) est réalisée de préférence à plusieurs reprises jusqu'à ce que tous les dispositifs de recouvrement (10-1,..., 10-5) utilisés soient enlevés des récipients de réaction respectifs, dans lequel les étapes sont réalisées de préférence par un dispositif de dosage automatisé, en particulier un robot de pipetage.

16. Dispositif de dosage, en particulier robot de pipetage, comprenant :
une plaque de support sur laquelle est positionné un empilement (60) de dispositifs de recouvrement (10-1,..., 10-5) non utilisés selon l'une des revendications 1 à 12 non utilisés et sur laquelle est positionné au moins un récipient de réaction (50), et
un dispositif de préhension, mobile dans trois directions principales par rapport à la plaque de support, qui est installé pour saisir des dispositifs de recouvrement (10-1,..., 10-5) de l'empilement et les poser sur le récipient de réaction (50), ainsi que pour enlever et évacuer du récipient de réaction (50) un dispositif de recouvrement (10-1,..., 10-5) utilisé.
